# EUROPEAN PATENT SPECIFICATION

(11) **EP 3 856 228 B1**
(45) Date of publication and mention of the grant of the patent: **08.03.2023**
(21) Application number: 19773104.5
(22) Date of filing: 26.09.2019
(51) Int. Cl.: A61K 38/19, A61K 9/70, A61K 31/573, A61K 35/761, A61K 47/68, A61P 17/02, A61P 17/10, A61P 17/06, A61P 17/00, A01K 67/027, A61K 9/00, A61K 45/06

(54) **TAFA4 POLYPEPTIDE FOR USE FOR REDUCING SKIN INFLAMMATION**
TAFA4 POLYPEPTID ZUR VERWENDUNG ZUR VERMINDERUNG VON HAUTENTZÜNDUNGEN
TAFA4 POLYPEPTIDE À UTILISER POUR RÉDUIRE L'INFLAMMATION DE LA PEAU

(30) Priority: 27.09.2018 EP 18306263
(43) Date of publication of application: 04.08.2021
(73) Proprietor: Institut National de la Santé et de la Recherche Médicale (INSERM), 75013 Paris (FR); Université d'Aix-Marseille, 13007 Marseille (FR); Centre National de la Recherche Scientifique (CNRS), 75016 Paris (FR)
(72) Inventor: UGOLINI, Sophie, 13288 MARSEILLE Cedex 09 (FR); DEBROAS, Guilhaume, 13288 MARSEILLE (FR); HOEFFEL, Guillaume, 13288 MARSEILLE CEDEX 09 (FR); MOQRICH, Abdelaziz, 13009 MARSEILLE (FR)
(74) Representative: Inserm Transfert
(86) International application number: PCT/EP2019/075987
(87) International publication number: WO 2020/064907

(56) References cited:
- WO-A2-2004/085469
- US-A1- 2016 113 996
- KAMBRUN CHARLINE ET AL: "TAFA4 Reverses Mechanical Allodynia through Activation of GABAergic Transmission and Microglial Process Retraction.", CELL REPORTS 13 MAR 2018, vol. 22, no. 11, 13 March 2018 (2018-03-13), pages 2886-2897, XP009511609, ISSN: 2211-1247

## Description

### FIELD:

The present disclosure is in the field of medicine, in particular skin inflammatory diseases.

### BACKGROUND:

The skin serves as an important boundary between the internal milieu and the environment, preventing contact with potentially harmful antigens. In the case of antigen/pathogen penetration, an inflammatory response is induced to eliminate the antigen. This response leads to a dermal infiltrate that consists predominantly of T cells, polymophonuclear cells, and macrophages Normally, this inflammatory response, triggered by the pathogen, is under tight control and will be halted upon elimination of the pathogen. In certain cases, the inflammatory response occurs in absence of pathogen. For instance, UV radiation causes sunburn-like damage characterized by the destruction of the epidermis and inflammation of the underneath dermal papilla ^{8,9}. These events lead to the rapid activation of mechanisms orchestrated by resident dermal macrophages and infiltrating monocytes to resolve inflammation and promote tissue repair ¹⁰. Excessive inflammation can however lead to chronic tissue damage followed by excessive collagen deposition resulting in unresolved fibrotic scars. Tissue-specific signals constantly shape resident macrophage functional identity ^{11,12 13} and promote their maintenance throughout the lifespan, either by local self-renewal or by the recruitment of additional monocyte-derived cells. However, the nature of these signals remains largely unknown.

TAFA4 is a member of the TAFA family which is composed of five highly homologous genes that encode small secreted proteins. These proteins contain conserved cysteine residues at fixed positions, and are distantly related to MIP-1alpha, a member of the CC-chemokine family. TAFA4 molecule is thus a chemokine-like protein ²³ known to modulates injury-induced mechanical and chemical pain hypersensitivity in mice ²². Recombinant TAFA4 was shown to be involved in human macrophage chemotaxis *in vitro* ²⁴. TAFA4 was also described as suitable for the treatment of pain (US20160113996). However, its potential role on myeloid cell recruitment or activation *in vivo* and eventually in skin inflammation has not yet been investigated.

### SUMMARY OF THE INVENTION:

The present invention relates to a TAFA4 polypeptide or a nucleic acid molecule encoding thereof for use for reducing skin inflammation in a subject in need thereof.

In particular, the present invention is defined by the claims.

### DETAILED DESCRIPTION:

The skin is one of first lines of defense against external threats. Tissue-resident macrophages have pivotal functions in tissue-barrier integrity and homeostasis. Their maintenance following injury, relies on tissue-specific signals controlling their self-renewal or their replacement by recruited circulating monocytes ¹. Upon skin infection or inflammation, a functional crosstalk between the sensory nervous system and tissue-resident immune cells can regulate cutaneous immune responses². However, depending on the pathological context, sensory neurons display pro- or anti-inflammatory regulatory properties ^{3 4 5 6} suggesting functional diversity within different sensory neuron subpopulations ⁷. Here the inventors identify, in a model of ultraviolet (UV)-induced skin damage, a regulatory role for type C low-threshold mechanoreceptor (C-LTMR) sensory neurons on the dynamic of dermal macrophage replacement by inflammatory monocytes through the neuropeptide TAFA4. *Tafa4*-KO mice present an unresolved fibrotic dermis after UV irradiation. Increased fibrotic score correlates with the upstream persistency of inflammatory monocytes and their MHC-II⁺ macrophage progeny. Bone marrow chimera revealed that inflammatory monocyte differentiation towards CD206+ dermal macrophage is increased in Tafa4KO recipient. Finally, intradermal injection of TAFA4 at the site of UV irradiation reduces inflammatory monocytes accumulation and skin inflammation in *Tafa4*-KO mice. The results provide new insight about tissue-resident macrophages dynamic during the resolution of skin fibrosis and thus renders credible the use of TAFA4 for the treatment of skin inflammation.

Accordingly, the first object of the present disclosure relates to a method of reducing skin inflammation in a subject in need thereof comprising administering to the subject a therapeutically effective amount of a TAFA4 polypeptide or a nucleic acid molecule encoding thereof.

The method of the present disclosure is thus particularly suitable for the treatment of skin inflammatory disease.

As used herein, the term "inflammatory skin disease" refers to diseases characterized by occurrence of a skin lesion resulting from infiltration of inflammatory cells such as activated helper T cells and monocytes.Examples of skin inflammatory diseases include, but are not limited to, acne, rosacea, folliculitis, perioral dermatitis, photodamage, skin aging, psoriasis, ichtiosis, chronic wounds, bed sores, keratosis piralis, scars, including surgical and acne scars, sebaceous cysts, inflammatory dermatoses, post inflammatory hyperpigmentation, xerosis, pruritis, lichen planus, nodular prurigo, eczema, and miliaria. Skin inflammatory diseases treatable in accordance with the disclosure also include, for example, chronic or acute skin inflammation, skin fibrosis, scleroderma, or a skin fibrotic disease or disorder.

In particular, the method herein disclosed is particularly suitable for the treatment of scleroderma, atopic dermatitis, nephrogenic fibrosing dermopathy, mixed connective tissue disease, scleromyxedema, scleredema, keloid, sclerodactyly, or eosinophilic fasciitis.

In particular, the method herein describes is particularly suitable for the treatment of photodermatitis. As used herein, the term "photodermatitis" has its general meaning in the art and refers to skin inflammation in response to UV radiation/light. This tissue response can include pain, irritation, itch, influx of inflammatory and pain-enhancing cells and tissue injury.

In particular, the method herein disclosed is particularly suitable for the treatment of chronic wound. As used herein, the term "chronic wound" refers to those wounds that do not heal in an orderly set of stages and in a predictable amount of time. Typically, wounds that do not heal within three months are considered chronic. Examples of chronic wounds include, but are not limited to, venous stasis ulcers, diabetic foot ulcers, and the like. For instance, for the treatment of diabetes and venous stasis ulcers, the scaffold is applied to the wounds to promote granulation tissue formation and facilitate epithelialization. Chronic, wounds may also include those relating to trauma (or repeated trauma), thermal injury (e.g., burns) and radiation damage. In particular, treatment of chronic wound in subject suffering from sickle-cell disease is also encompassed. In particular, treatment of chronic wound in elderly is also encompassed.

In particular, the method herein disclosed is particularly suitable for preventing skin fibrosis. As used herein, the term "skin fibrosis" or "dermal fibrosis" means the excessive proliferation of epithelial cells or fibrous connective tissue (fibrosis) thereby resulting in the development of scarred (fibrotic) tissue. The scarred tissue replaces healthy tissue by the process of fibrosis and may be the prelude of systemic scleroderma.

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein, the term "TAFA4 polypeptide" designates a polypeptide belonging to the family of TAFA chemokine-like proteins, preferably comprising the amino acid sequence of SEQ ID NO: 1 (which corresponds to the human TAFA4 amino acid sequence) and any natural variant thereof (e.g., variants present in other animal species, or variants as a result of polymorphism or splicing). Within the context of the present disclosure, the term "TAFA4 polypeptide" also includes any polypeptide comprising a sequence having at least 90% sequence identity to the sequence shown in SEQ ID NO: 1.

SEQ ID NO:1 >sp|Q96LR4|F19A4_HUMAN Protein FAM19A4 OS=Homo sapiens
OX=9606 GN=FAM19A4 PE=1 SV=1
MRSPRMRVCAKSVLLSHWLFLAYVLMVCCKLMSASSQHLRGHAGHHQIKQGTCEVVAVHR
CCNKNRIEERSQTVKCSCFPGQVAGTTRAQPSCVEASIVIQKWWCHMNPCLEGEDCKVLP
DYSGWSCSSGNKVKTTKVTR

According to the disclosure a first amino acid sequence having at least 90% of identity with a second amino acid sequence means that the first sequence has 90; 91; 92; 93; 94; 95; 96; 97; 98; 99 or 100% of identity with the second amino acid sequence. Sequence identity is frequently measured in terms of percentage identity (or similarity or homology); the higher the percentage, the more similar are the two sequences. Methods of alignment of sequences for comparison are well known in the art. Various programs and alignment algorithms are described in: Smith and Waterman, Adv. Appl. Math., 2:482, 1981; Needleman and Wunsch, J. Mol. Biol., 48:443, 1970; Pearson and Lipman, Proc. Natl. Acad. Sci. U.S.A., 85:2444, 1988; Higgins and Sharp, Gene, 73:237-244, 1988; Higgins and Sharp, CABIOS, 5:151-153, 1989; Corpet et al. Nuc. Acids Res., 16:10881-10890, 1988; Huang et al., Comp. Appls Biosci., 8:155-165, 1992; and Pearson et al., Meth. Mol. Biol., 24:307-31, 1994). Altschul et al., Nat. Genet., 6:119-129, 1994, presents a detailed consideration of sequence alignment methods and homology calculations. By way of example, the alignment tools ALIGN (Myers and Miller, CABIOS 4:11-17, 1989) or LFASTA (Pearson and Lipman, 1988) may be used to perform sequence comparisons (Internet Program^{®} 1996, W. R. Pearson and the University of Virginia, fasta20u63 version 2.0u63, release date December 1996). ALIGN compares entire sequences against one another, while LFASTA compares regions of local similarity. These alignment tools and their respective tutorials are available on the Internet at the NCSA Website, for instance. Alternatively, for comparisons of amino acid sequences of greater than about 30 amino acids, the Blast 2 sequences function can be employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). The BLAST sequence comparison system is available, for instance, from the NCBI web site; see also Altschul et al., J. Mol. Biol., 215:403-410, 1990; Gish. & States, Nature Genet., 3:266-272, 1993; Madden et al. Meth. Enzymol., 266:131-141, 1996; Altschul et al., Nucleic Acids Res., 25:3389-3402, 1997; and Zhang & Madden, Genome Res., 7:649-656, 1997.

The polypeptides of the disclosure may be produced by any suitable means, as will be apparent to those of skill in the art. In order to produce sufficient amounts of polypeptides or functional equivalents thereof for use in accordance with the present disclosure, expression may conveniently be achieved by culturing under appropriate conditions recombinant host cells containing the polypeptide of the disclosure. In particular, the polypeptide is produced by recombinant means, by expression from an encoding nucleic acid molecule. Systems for cloning and expression of a polypeptide in a variety of different host cells are well known. When expressed in recombinant form, the polypeptide is in particular generated by expression from an encoding nucleic acid in a host cell. Any host cell may be used, depending upon the individual requirements of a particular system. Suitable host cells include bacteria mammalian cells, plant cells, yeast and baculovirus systems. Mammalian cell lines available in the art for expression of a heterologous polypeptide include Chinese hamster ovary cells. HeLa cells, baby hamster kidney cells and many others. Bacteria are also preferred hosts for the production of recombinant protein, due to the ease with which bacteria may be manipulated and grown. A common, preferred bacterial host is E coli.

In particular, the TAFA4 polypeptide is fused to an immunoglobulin constant domain to constitute an immunoadhesin. As used herein, the term "immunoadhesin" designates antibody-like molecules which combine the binding specificity of a heterologous protein (an "adhesion") with the effector functions of immunoglobulin constant domains. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM. The immunoglobulin sequence typically, but not necessarily, is an immunoglobulin constant domain (Fc region). Immunoadhesins can possess many of the valuable chemical and biological properties of human antibodies. Since immunoadhesins can be constructed from a human protein sequence with a desired specificity linked to an appropriate human immunoglobulin hinge and constant domain (Fc) sequence, the binding specificity of interest can be achieved using entirely human components. Such immunoadhesins are minimally immunogenic to the patient, and are safe for chronic or repeated use.

The polypeptides of the disclosure can exhibit post-translational modifications, including, but not limited to glycosylations, (e.g., N-linked or O-linked glycosylations), myristylations, palmitylations, acetylations and phosphorylations (e.g., serine/threonine or tyrosine).

In particular, it is contemplated that polypeptides used in the therapeutic methods of the present disclosure may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution. In example adding dipeptides can improve the penetration of a circulating agent in the eye through the blood retinal barrier by using endogenous transporters.

As used herein, the term "nucleic acid molecule" has its general meaning in the art and refers to a DNA or RNA molecule. However, the term captures sequences that include any of the known base analogues of DNA and RNA such as, but not limited to 4-acetylcytosine, 8-hydroxy-N6-methyladenosine, aziridinylcytosine, pseudoisocytosine, 5-(carboxyhydroxylmethyl) uracil, 5-fiuorouracil, 5-bromouracil, 5-carboxymethylaminomethyl-2-thiouracil, 5-carboxymethyl-aminomethyluracil, dihydrouracil, inosine, N6-isopentenyladenine, 1 -methyladenine, 1 -methylpseudouracil, 1-methylguanine, 1- methylinosine, 2,2-dimethylguanine, 2-methyladenine, 2-methylguanine, 3-methylcytosine, 5- methylcytosine, N6-methyladenine, 7-methylguanine, 5-methylaminomethyluracil, 5-methoxyamino-methyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, -uracil-5-oxyacetic acid methylester, uracil-5-oxyacetic acid, pseudouracil, queosine, 2-thiocytosine, and 2,6-diaminopurine.

In particular, the nucleic acid molecule of the present disclosure comprises a nucleic acid sequence having has at least 70% identity with the nucleic acid sequence as set forth in SEQ ID NO:2. According to the disclosure a first nucleic acid sequence having at least 70% identity with a second nucleic acid sequence means that the first sequence has 70; 71; 72; 73; 74; 75; 76; 77; 78; 79; 80; 81; 82; 83; 84; 85; 86; 87; 88; 89; 90; 91; 92; 93; 94; 95; 96; 97; 98; 99 or 100% identity with the second nucleic acid sequence.

SEQ ID NO:2

1 aatcagcgcg ttccgcggcg ggcccggccc ctcctggtca gcgcgctagc tgggctcggc
61 tccgcactgc tagctgcgcg ccgccctgga cggggcgcac cgactgcgcg cgcggctgcg
121 ggcaaacatc gggagtcctg cctcagctgc cgcttctcca gcagcagctt caggcttctc
181 ccgcaggagc ttcgggcttc tcctggtaga gacgtgggaa cttttcttct cctggcgagg
241 ctgcagaggt gatgggccgc tcccggggct cccgcgggga ggcggcacgg tgagcgtcct
301 cgggctccgg tgcggcgatc agtacctagt tccggacgcg ccggtccgac ttggatgccg
361 gctctagtcg agtcttctga gctacgataa ttttttggaa cggcagaaat gattggttct
421 agcaacagat gggaatttgg agtcactctg aaatatatcc tggaataagt gtgtttgact
481 agaaccacat cttatgaggt ccccaaggat gagagtctgt gctaagtcag tgttgctgtc
541 gcactggctc tttctagcct acgtgttaat ggtgtgctgt aagctgatgt ccgcctcaag
601 ccagcacctc cggggacatg caggtcacca ccaaatcaag caagggacct gtgaggtggt
661 cgccgtgcac aggtgctgca ataagaaccg catagaagag cggtcacaaa cggtcaagtg
721 ctcttgcttc ccgggacagg tggcgggcac aactcgggct caaccttctt gtgttgaagc
781 ttccattgtg attcagaaat ggtggtgtca catgaatccg tgtttggaag gagaggattg
841 taaagtgctg ccagattact caggttggtc ctgtagcagt ggcaataaag tcaaaactac
901 gaaggtaacg cggtagcgaa gagagaggtg tgcttcaatc ctggaggggc agcaggaggc
961 ggagctcttt tgcttggatt cccatcatgg cccctttgca gaaaattgtc taggatttca
1021 gcaacttcat atttgtatat gtgagctgtg agaggtggca ttcacttaac tggcccagcc
1081 ctctctgctt cgtgatttta tttcattgaa ttataaccac aagccaccac ccatttgaca
1141 tcctctctgg attcccaagg agcatacctc caaaatccga gaagagcaaa tcagagtctt
1201 caaaatggat caccactaag ggcatgttca ttcttcactt tctttctgct tttacaaaag
1261 aacttggatg tatgttccaa agggtcctca ttctgttcct cttttgaact tttccttttg
1321 tccttgtatt aaagtggttt taaaggggtc taaaaagatt ttggcaaaac atatttgcag
1381 atgtagatta gctggtgaag aaaattactg ctagagatca actgattaac tggtaaagaa
1441 cgtttatttt ataacccttg aagaatagaa ggacatagtt ggattattgt gtgtgcattg
1501 tatttttact tctatttttt ttttgctttc cattttccag ttagcagaga taaaatgaga
1561 gcgttttaac ttcaatgtac cattttactg agtgctaagg aagcatatca attccaatat
1621 tttataacca aagctctatc agaacatatt tataaaactt gttggaattt ttacggcttt
1681 tgtgtagtca tgtaggtaaa tcatttaaaa tataaaacaa tctcaattta gatcaagggt
1741 tatttcttag atcaaattta tgccaattat atgaaaagat tttaactccg agacaggagt
1801 ctttcagtgc tgaattttta gactgtaaat gagttcttct taacttagct gtttccctac
1861 ttctgtgact tctgtgttag ccatcttatt tctttaaaat ctgagtcctg attggcttaa

1921 tgattttgca gcagacatgt ctccacatat tctcaaatgc tgtcatgcgg aaacgtatga
1981 aacagatgaa gaatgactga cccagatttt agatgtataa tgttgttaaa gtacatacta
2041 ctgtaaaaat atgggatgaa ttttatatat taagaaatgc caaaaacata gtttctgcac
2101 caagttaatt atccctgtcc tttcacattt atagggggaa aataaatact ttaatgttgt
2161 ttatagccta acagttattt gattttattc ttgcagaggg aatggaaagg aatggaaaga
2221 tttgttggcg taatttttga atatttgtta tgatcatatg aataagtaaa aaaattcatc
2281 ctgctgatgg cata

In particular, the nucleic acid molecule of the present disclosure is included in a suitable vector. Typically, the vector is a viral vector, and more particularly an adeno-associated virus (AAV), a retrovirus, bovine papilloma virus, an adenovirus vector, a lentiviral vector, a vaccinia virus, a polyoma virus, or an infective virus. In particular, the vector is an AAV vector. As used herein, the term "AAV vector" means a vector derived from an adeno- associated virus serotype, including without limitation, AAV1, AAV2, AAV3, AAV4, AAV5, AAV6, AAV7, AAV8, AAV9, and mutated forms thereof. AAV vectors can have one or more of the AAV wild-type genes deleted in whole or part, preferably the rep and/or cap genes, but retain functional flanking ITR sequences. Retroviruses may be chosen as gene delivery vectors due to their ability to integrate their genes into the host genome, transferring a large amount of foreign genetic material, infecting a broad spectrum of species and cell types and for being packaged in special cell- lines. In order to construct a retroviral vector, a nucleic acid encoding a gene of interest is inserted into the viral genome in the place of certain viral sequences to produce a virus that is replication-defective. In order to produce virions, a packaging cell line is constructed containing the gag, pol, and/or env genes but without the LTR and/or packaging components. When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviral vectors are able to infect a broad variety of cell types. Lentiviruses are complex retroviruses, which, in addition to the common retroviral genes gag, pol, and env, contain other genes with regulatory or structural function. The higher complexity enables the virus to modulate its life cycle, as in the course of latent infection. Some examples of lentivirus include the Human Immunodeficiency Viruses (HIV 1, HIV 2) and the Simian Immunodeficiency Virus (SIV). Lentiviral vectors have been generated by multiply attenuating the HIV virulence genes, for example, the genes env, vif, vpr, vpu and nef are deleted making the vector biologically safe. Lentiviral vectors are known in the art, see, e.g.. U.S. Pat. Nos. 6,013,516 and 5,994,136,. In general, the vectors are plasmid-based or virus-based, and are configured to carry the essential sequences for incorporating foreign nucleic acid, for selection and for transfer of the nucleic acid into a host cell. The gag, pol and env genes of the vectors of interest also are known in the art. Thus, the relevant genes are cloned into the selected vector and then used to transform the target cell of interest. Recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136. This describes a first vector that can provide a nucleic acid encoding a viral gag and a pol gene and another vector that can provide a nucleic acid encoding a viral env to produce a packaging cell. Introducing a vector providing a heterologous gene into that packaging cell yields a producer cell which releases infectious viral particles carrying the foreign gene of interest. The env preferably is an amphotropic envelope protein which allows transduction of cells of human and other species. Typically, the nucleic acid molecule or the vector of the present disclosure include "control sequences'", which refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell. Another nucleic acid sequence, is a "promoter" sequence, which is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. Transcription promoters can include "inducible promoters" (where expression of a polynucleotide sequence operably linked to the promoter is induced by an analyte, cofactor, regulatory protein, etc.), "repressible promoters" (where expression of a polynucleotide sequence operably linked to the promoter is induced by an analyte, cofactor, regulatory protein, etc.), and "constitutive promoters".

By a "therapeutically effective amount" is meant a sufficient amount of the polypeptide or the nucleic acid molecule encoding thereof to prevent for use in a method for the treatment of the skin inflammatory disease at a reasonable benefit/risk ratio applicable to any medical treatment. It will be understood that the total daily usage of the compounds and compositions of the present disclosure will be decided by the attending physician within the scope of sound medical judgment. The specific therapeutically effective dose level for any particular subject will depend upon a variety of factors including the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

According to the disclosure, the polypeptide or the nucleic acid molecule of the present disclosure is administered to the subject in the form of a pharmaceutical composition. Typically, the polypeptide or the nucleic acid molecule (inserted or not into a vector) of the present disclosure may be combined with pharmaceutically acceptable excipients, and optionally sustained-release matrices, such as biodegradable polymers, to form pharmaceutical compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present disclosure for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, intradermal, transdermal, intrathecal and intranasal administration forms and rectal administration forms.

In particular, the pharmaceutical compositions are formulated for topical administration. The topical pharmaceutically acceptable carrier is any substantially nontoxic carrier conventionally usable for topical administration of pharmaceuticals in which the active ingredient of the disclosure will remain stable and bioavailable when applied directly to skin. For example, carriers such as those known in the art effective for penetrating the keratin layer of the skin into the stratum comeum may be useful in delivering the active ingredient of the disclosure to the area of interest. Such carriers include liposomes. The active ingredient of the disclosure can be dispersed or emulsified in a medium in a conventional manner to form a liquid preparation or mixed with a semi-solid (gel) or solid carrier to form a paste, powder, ointment, cream, lotion or the like. Suitable topical pharmaceutically acceptable carriers include water, buffered saline, petroleum jelly (vaseline), petrolatum, mineral oil, vegetable oil, animal oil, organic and inorganic waxes, such as microcrystalline, paraffin and ozocerite wax, natural polymers, such as xanthanes, gelatin, cellulose, collagen, starch, or gum arabic, synthetic polymers, alcohols, polyols, and the like. The carrier can be a water miscible carrier composition. Such water miscible, topical pharmaceutically acceptable carrier composition can include those made with one or more appropriate ingredients outset of therapy. Because dermatologic conditions to be treated may be visible, the topical carrier can also be a topical cosmetically acceptable carrier. The topical cosmetically acceptable carrier will be any substantially non-toxic carrier conventionally usable for topical administration of cosmetics in which active ingredient of the disclosure will remain stable and bioavailable when applied directly to the skin surface. Suitable cosmetically acceptable carriers are known to those of skill in the art and include, but are not limited to, cosmetically acceptable liquids, creams, oils, lotions, ointments, gels, or solids, such as conventional cosmetic night creams, foundation creams, suntan lotions, sunscreens, hand lotions, make-up and make-up bases, masks and the like. Topical cosmetically acceptable carriers may be similar or identical in nature to the above described topical pharmaceutically acceptable carriers. The compositions can contain other ingredients conventional in cosmetics including perfumes, estrogen, vitamins A, C or E, alpha-hydroxy or alpha-keto acids such as pyruvic, lactic or glycolic acids, lanolin, vaseline, aloe vera, methyl or propyl paraben, pigments and the like.

It may be desirable to have a delivery system that controls the release of active ingredient of the disclosure to the skin and adheres to or maintains itself on the wound for an extended period of time to increase the contact time of the active ingredient of the disclosure on the skin. Sustained or delayed release of active ingredient of the disclosure provides a more efficient administration resulting in less frequent and/or decreased dosage of active ingredient of the disclosure and better patient compliance. Examples of suitable carriers for sustained or delayed release in a moist environment include gelatin, gum arabic, xanthane polymers. Pharmaceutical carriers capable of releasing the active ingredient of the disclosure when exposed to any oily, fatty, waxy, or moist environment on the area being treated, include thermoplastic or flexible thermoset resin or elastomer including thermoplastic resins such as polyvinyl halides, polyvinyl esters, polyvinylidene halides and halogenated polyolefins, elastomers such as brasiliensis, polydienes, and halogenated natural and synthetic rubbers, and flexible thermoset resins such as polyurethanes, epoxy resins and the like. Controlled delivery systems are described, for example, in U.S. Pat. No. 5,427,778 which provides gel formulations and viscous solutions for delivery of the active ingredient of the disclosure to a skin site. Gels have the advantages of having a high water content to keep the skin moist, the ability to absorb skin exudate, easy application and easy removal by washing. Preferably, the sustained or delayed release carrier is a gel, liposome, microsponge or microsphere.

In particular, topical administration for cutaneous treatment is accomplished via a transdermal device or "patch" device. The active ingredient of the disclosure formulations for transdermal administration can be used to coat the fibers of an absorbent gauze dressing.

In particular, the TAFA4 polypeptide is administered in combination with classical treatment of skin inflammation.

As used herein, the term "classical treatment" refers to any active agent used for the treatment of skin inflammation.

In particular, the classical treatment of skin inflammation refers to calcineurin inhibitor such as tacrolimus and pimecrolimus; dapsone; retinoids; methotrexate; and glucocorticoid.

In particular, the TAFA4 polypeptide is administered in combination with another active agent such as a glucocorticoid. Specific non-limiting examples of glucocorticoids include dexamethasone, triamcinolone acetonide (AZMACORT^{®}), beclomethasone, dipropionate (VANCERIL^{®}), flunisolide (AEROBID^{®}), fluticasone propionate (FLOVENT^{®}), prednisone, methylprednisolone and mometasonefuroate (ASMANEX^{®}, TWISTHALER^{®}).

As used herein, the terms "combined treatment", "combined preparation", "combined therapy" or "therapy combination" refer to a treatment that uses more than one medication. The combined therapy may be dual therapy or bi-therapy.

The medications used in the combined treatment according to the disclosure are administered to the subject simultaneously, separately or sequentially.

As used herein, the term "administration simultaneously" refers to administration of 2 active ingredients by the same route and at the same time or at substantially the same time. The term "administration separately" refers to an administration of 2 active ingredients at the same time or at substantially the same time by different routes. The term "administration sequentially" refers to an administration of 2 active ingredients at different times, the administration route being identical or different.

The disclosure will be further illustrated by the following figures and examples. However, these examples and figures should not be interpreted in any way as limiting the scope of the present disclosure.

### FIGURES:

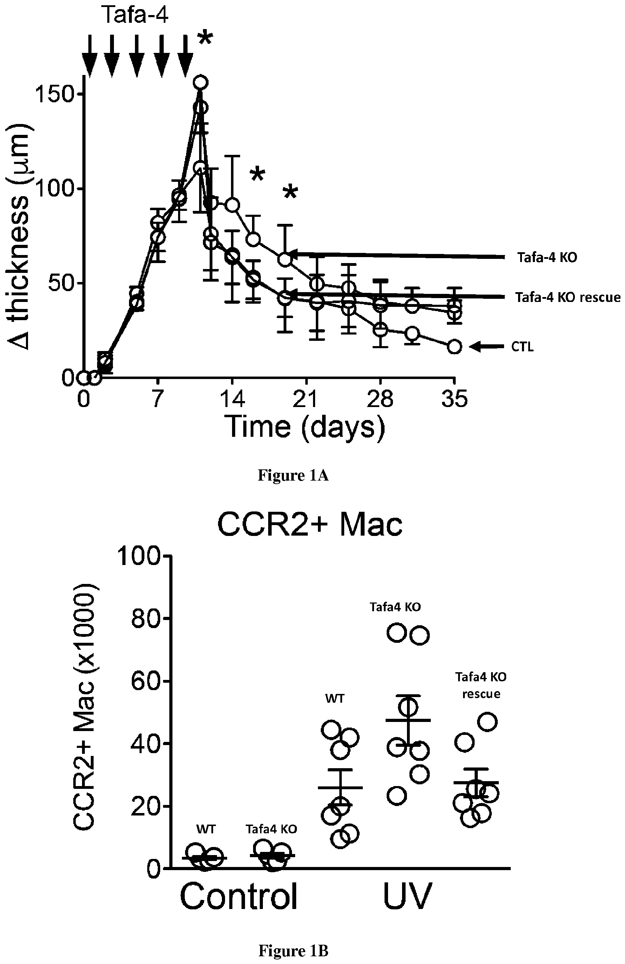

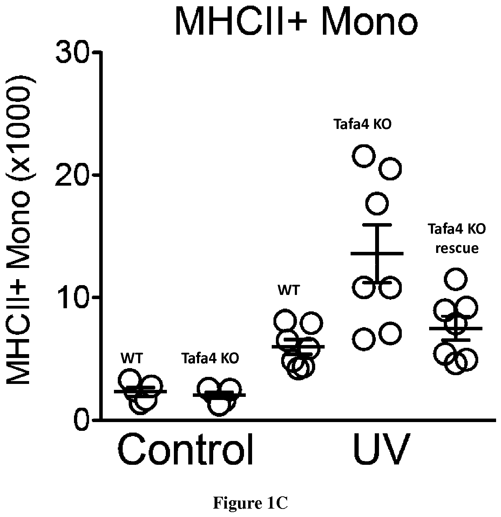

Figure 1: (A) WT (CTL), *Tafa4*-KO mice were treated with UV and ear thickness was followed over time. *Tafa4*-KO mice were treated either with saline (Tafa-4 KO or recombinant TAFA4 protein (100nM) (Tafa-4 KO rescue). N=10 mice per group. (B-C) Absolute number of CCR2⁺ myeloid cell (B) or MHC-II⁺ macrophages (C) was assessed at day 7 post-UV treatment. All data are represented as mean+/-SEM. (*p < 0.05; **p < 0.01; ***p < 0.001).

### EXAMPLE:

### Material & Methods

### Mice

Mice were maintained under standard housing conditions with free pathogen, free access to food and water and a 12h light and dark cycle at an ambiant temperature of 22°C in the animal facility of the CIML or CIPHE (CIML, France). C57/Bl6J mice were bought from Janvier (https://www.janvier-labs.com) and TAFA4-KO and GINIP-DTR mice were born in our animal house. TAFA-4-KO mice and NaV1.8CRE-GINIP-DTR mice were generated by Aziz Moqrich's team (IBDM, AMU, France) and described previously ^{17,22}. CCR2-KO mice were described previously ³¹. Special effort was made to minimize number and stress. All protocols are in agreement with European Union recommendations for animal experimentation. All mice were used between 8 and 12 weeks unless specified.

### Bone Marrow Chimera generation

Age and sex matched WT or TAFA4KO mice were anaesthetized with ketamine/xylazine (10/µl/g, 2% Imalgene500 et 5% Rompun). Then mice were lethally irradiated with 6,5Gy from an X-ray irradiator. A lead shield was used for protecting the ear's skin from the irradatiation-induced damage. 6h after, 30mg/kg busulfan was injected (i.p.) to deplete remaining myeloid progenitors in recipient mice. 12h after these mice were reconstituted with 5-10 million bone marrow cells from CD45.1 or CCR2-KO mice. After irradiation, mice were treated with bactrim (in drinking water) for two weeks for a total recovery time of 5 weeks before analyzing the chimerisms efficiency and used for experiments.

### Skin injury

Age and sex matched WT, TAFA4KO or GINIP-DTR mice were anaesthetized with ketamine/xylazine (10/µl/g, 2% Imalgene500 et 5% Rompun) and then left untreated or exposed to UV (wavelength: 254nm ; voltage : 8W ; source : 30cm from the target) for 30 min as previously described ⁴². Ear thickness was followed every other day with a caliper (mice placed under isofluorane anesthesia) and assessed as thickness of UV-treated ears minus thickness of controls untreated ears.

### Histology and anatomopathology analysis

At different time after UV treatment, mice were lethally anesthetized ketamine/xylazine (15µl/g, 20% Imalgene500 et 5% Rompun) then 100ml of blood was collected with glass Pasteur pipette. Mice were then perfused with 10ml of PBS. Ears were then collected and fixed with formol (for at least 1h then cryoprotected with sucrose 30% overnight and embedded in paraffin. Skin sections of 5µm were stained with haematoxylin and eosin or picrosirius red. Histological scores were blinded assessed by a pathologist following the criteria described in table 2S. Briefly grading of inflammation, of wound healing and fibrosis with a semiquantitative evaluation of fibrosis amount on one section and of the thickness of epidermis was made.

### Dorsal Root Ganglia (DRG) isolation and tissue section

After anesthesia (ketamine/xylazine mix in i.p.: 15µl/g mice, 5% Rompun at 2% + 20% Imalgen 500), mice were injected with 5ml of cold PBS and right after with 25ml of AntigenFix (PFA 4%) (i.c.). DRG were carefully dissected from the spinal cord under a binocular and collected in AntigenFix (PFA 4%). Then DRG were post-fixed in AntigenFix (PFA 4%) for at least 1h then cryoprotected with 30% (w/v) sucrose overnight before being frozen in OCT and stored at -80°C. DRG samples were sectioned at 12µm using a standard cryostat (Leica).

### In situ Hybridization and immunofluorescence on DRG

In situ hybridization was carried out following protocol from ¹⁶. Briefly, RNA probes were synthesized using gene-specific PCR primers and cDNA templates from mouse DRG.

DRG sections were treated with proteinase K then thriethanolamine and acetic anhydride solutions. Digoxigenin labeled probes were hybridized overnight at 55°C after 2h of prehybridization. The slides were treated with 0.2X SSC baths then blocked at room temperature with 10% Goat serum and incubated with anti-digoxigenin antibody (Roche). Final detection was achieved using Cyanine 3 TSA plus kit (Perkin Elmer).

For immunofluorescence, DRG sections were permeabilized with 0.3% Triton X-100 in FACS buffer for 1h at room temperature. Primary antibodies were incubated as follows: rabbit anti-ATF3 1:200 (Santa Cruz SC-188), rat anti-TAFA4 (clone 1D8 1:500; MiMabs) overnight at 4°C, rat anti-Ginip 1:1000 (generous gift from Dr. A. Moqrich, IBDMI,) overnight at 4°C, goat anti-CGRP 1:1000 (Abeam Ab36000) 2h at 37°C. Corresponding secondary antibodies (Alexa405, 488 or 594; Jackson ImmunoReasarch, 712-585-153, 712-475-153, 711-545-152, 705-585-147) were incubated 45 minutes at room temperature. Isolectin B4 conjugates with AlexaFluor 647 dye (Invitrogen) was used at 1:200, 45 minutes at room temperature.

Acquisition of images was performed on Confocal LSM780 (Zeiss) and analyzed with ZEN and ImageJ software.

### Skin cells isolation

After UV treatment, mice were euthanized. Ears were split into dorsal and ventral layer then minced into small pieces and incubated 1h at 37°C in complete medium (RPMI+L-Glutamine 10% SVF) auditioned with 1mg/ml DNase (Roche) 0.2ml/ml Dispase (GIBCO) and 0.2mg/ml collagenase type IV (Sigma). Then tissues were dissociated using 2.5 ml syringes and 18G needles and filtered on cell strainer (100mm, BD), washed with Facs buffer (PBS-2mM EDTA, FCS 2%, BSA 0.5%) to obtain a homogeneous cell suspension ready for staining.

### Antibody

Single-cell suspension were plated in 96 well U bottom plates and stained at 4°C. Dead cells were removed by counter-gating on the Livedead fixable blue Dead Cell Stain kit UV (L23105; Invitrogen). Antibodies used: CD45-BV785 (30F11; Biolegend), CD45.1-BV605 (A20; Biolegend), CX3CR1-BV421 (SA011F1), CD11b-BV510 (M1/70; BD Biosciences), CD64-BV711 (X54-5/7.1; Biolegend), CD169-BV605 (3D6.112; Biolegend), Ly6C-FITC (AL-21; BD Biosciences), F4-80-PECF594 (T45-2342; BD Biosciences), CD11c-PECy7 (N418; Biolegend), CCR2-PE (475301; R&D System), SiglecF-PE (E50-2440; BD Biosciences), CD206-APC (C068C2; Biolegend), Ly6G-APC-Cy7 (1A8; Biolegend), IA-IE-A700 (M5/114.15.2; Biolegend), CD24-BUV395 (M1/69; BD Biosciences), CD103-biotin (M290; BD Bioscience), MerTK-biotin (polyclonal, BAF591 R&D System), IL-1b-PE Ab (BD) anti-TNFa-PE (BD) Streptavidin-BUV737 (BD Biosciences).

### Flow cytometry

Cells were incubated 40min at 4°C in Facs buffer with antibody + 2.4 G2 antibody to block Fc receptors, washed and fixed until analysis. For ex-vivo cytokines staining, cells suspensions were permeabilized using (fixperm FoxP3 kit; ebioscience) and stained with anti-IL-1b and TNF-a antibody. Multiparameter FACs analysis was performed using an LSR X20 system (BD). Absolute numbers for each population were obtained using Quanti Beads (556296, BD Bioscience). FACs analysis was performed using Flowjo software (Tree Star, Inc.).

### Bone marrow derived macrophage generation and in vivo treatment

Femurs and tibia bone marrow cells were flushed with sterile PBS, filtered (cell strainer, 100mm, BD) and then cultured in complete DMEM (DMEM, SVF 10%, P/S, 1% L-Glu) supplemented with 10% L929 cell-conditioned medium. After 7 days in culture, mature BMDMs were harvested by washing with cold PBS, incubating on ice for 15 min, and pipetting extensively. 1 × 106 BMDMs/well were plated onto 12-well plates (BD) in complete DMEM medium supplemented with 100ng.mL M-CSF) and incubated with alternatively activated macrophage-inducing stimuli (10ng.mL IL-4 Peprotech) with or without 10 or 100nM TAFA-4 (R&D System). After 16 hours, BMDM were lysed using RLT medium (Qiagen) then pass through a QIAShredder column (Qiagen) for homogenysation and store at -80°C.

### Thyoglycolate generated macrophage and in vitro treatment

Peritoneal macrophages were isolated from C57Bl/6J (aged 8 to 12 weeks) from a peritoneal wash three days after 3% thyoglycolate injection i.p. (Sigma). Cells were seeded at 1x106 cells per well in complete DMEM (DMEM, FCS 10%, P/S, 1% L-glu) and incubated with or without LPS (100ng.ml, Sigma) in presence of increasing concentration of TAFA-4 (R&D System ; 0, 1, 10, 100 or 1000nM). After 8 hours, macrophages were lysed with a lysing medium (RLT, QIAGEN) then centrifuged in QIAShredder column for homogeinisation to preserve nucleic acid which were conserved at -80°C.

### Gene expression analysis

Total RNA was isolated from ears untreated or treated with UV irradiation. Ears were dilacerated by FastPrep-24 (MpBio) with matrix A beads (MpBio). RNA was isolated using a fibrous RNeasy minikit (QIAGEN). Reverse transcription was performed using Superscript RTII (Invitrogen). Preamplification was performed using Taqman probe for gene target with pre-amplification master mix (Fluidigm). Pre-amplified products (18 cycles) were diluted five times before analysis with Universal PCR Master Mix and Taqman gene expression assays in 96.96 Dynamic Arrays on a BioMark System (Fluidigm). Data were analysed on the BioMark Real-Time PCR Analysis (Fluidigm) and normalised on a housekeeping gene, GADPH for in vitro experiment and HPRT for in vivo experiment (2^-ΔCt). All Taqman gene were bought from Applied Biosystems and were the recommended one by the seller.

### Statistical analysis

Results are expressed as means+/- SEM. Statistical analysis was performed using the GraphPad Prism for Windows software. Statistical significance of the data was compared using the student's t test or Mann and Witney test if 2 group were compared. If multiple group, 2-way annova or multiple t test with bonferroni correction were used. Difference were considered significant as following: *p<0.05; **p<0.01; ***p<0.001; and ****p<0.0001.

### Results and discussion:

UV radiation causes sunburn-like damage characterized by the destruction of the epidermis and inflammation of the underneath dermal papilla ^{8,9}. These events lead to the rapid activation of mechanisms orchestrated by resident dermal macrophages and infiltrating monocytes to resolve inflammation and promote tissue repair ¹⁰. Excessive inflammation can however lead to chronic tissue damage followed by excessive collagen deposition resulting in unresolved fibrotic scars. Tissue-specific signals constantly shape resident macrophage functional identity ^{11,12 13} and promote their maintenance throughout the lifespan, either by local self-renewal or by the recruitment of additional monocyte-derived cells. However, the nature of these signals remains largely unknown. We hypothesized that the highly developed sensory neuron network present within the skin could provide key signals required for myeloid cell functions in tissue repair.

To understand how this potential neuro-immune crosstalk could occur after skin injury, we exposed the ears of wild type (WT) mice to UV-C irradiation to induce skin damage in sterile conditions (**data not shown**). The skin has a highly developed sensory nervous system that detects external stimuli and tissue lesions. Upon UV exposure, sunburned areas are generally characterized by a temporal phase of hypersensitivity to pain mediated by the activation of specialized sensory neurons innervating the skin ¹⁴. We analyzed the kinetic of activation of cutaneous neurons after UV exposure. First, we traced sensory neurons innervating the ears by injecting the fluorescent dye DiI intradermally in the ear and analyzing the dorsal root ganglia (DRG) neurons for the presence of the dye within their cell body by confocal microscopy (**data not shown**). We analyzed the C2, C3, C4 DRGs and the trigeminal ganglia (TG) and found that the C2 and C3 were the DRG harboring the higher level of DiI staining showing that these DRG were the ones innervating the ears. The induction of the activating transcription factor 3 (ATF3) in DRG neurons was previously shown as a sensitive cellular marker to reveal the primary afferent fibers that are engaged by diverse chemical or noxious stimuli ¹⁵. We investigated the capacity of skin innervating neurons to react to UV exposure by monitoring the expression profile of *Atf3* by qRT-PCR in pooled C2/C3 DRGs over a time course of 14 days post-UV treatment (**data not shown**). We observed that, compared to non-exposed mice, UV-irradiation induced a significant expression of ATF3 mRNA in DRGs within one day. The maximal expression of *Atf3* was reached by day 3 and maintained at a high level at least until day 7 post UV-irradiation. Residual expression of *Atf3* gene was still observed at day 14 post-UV exposure. We then followed the kinetic of skin inflammation in our model by analyzing in the skin the expression profile of genes involved in myeloid cell activation, inflammation and tissue repair, at different time point post-irradiation by qRT-PCR (**data not shown**)**.** This analysis revealed that inflammatory genes encoding factors such as IL1β, TNF-α, CXCL2, CCL2, CCL3, CCL6 and CXCL10 were up-regulated between day 3 and day 7 post-UV irradiation highlighting the inflammatory phase induced by UV. The expression of these genes slowly decreased afterwards, while the expression of genes involved in tissue repair such as *Col1a* and *RETLNa* raised only after day 14 until at least day 35, highlighting the phase of recovery and the onset of tissue remodeling. These data suggest that the kinetic of activation of sensory neurons within DRG correlates with the degree of inflammation observed within the skin exposed to UV (**data not shown**).

Upon skin invasion with pathogens or exposure to chemicals the deletion of subsets of skin sensory neurons can be either protective or deleterious for host defense and were found to induce either pro- or anti-inflammatory responses ^{3 4 5 6}. The variety of functional outcomes observed upon sensory neuron deficiencies ² could be explained by the complexity and diversity of sensory neurons subtypes ⁷ bur their respective role in controlling immune cell functions and the molecular basis involved remain unclear.

To delineate the phenotype of the sensory neurons activated by UV irradiation, we performed immunofluorescent staining and confocal microcopy analysis of C3 DRGs of WT mice exposed or not to UV. At day 3 post-UV, ATF3 staining was observed in the nucleus of a subset of sensory neurons expressing the Gαi-Interacting Protein (GINIP) in exposed mice but not in control conditions (**data not shown**). Interestingly, this translocation of ATF3 was not observed in CGRP⁺ peptidergic neurons in UV-exposed mice (**data not shown**). These data suggest that GINIP⁺ neurons were preferentially activated by UV treatment. As their potential role on the control of cutaneous inflammation was not addressed so far, we decided to dissect their specific role in tissue repair in the context of UV irradiation-induced skin damage. We used a genetic model allowing the tissue specific and inducible ablation of GINIP-expressing neurons ¹⁶. This model was obtained by crossing GINIP^{flx/+} line ¹⁷ with mice expressing the CRE recombinase from *Nav1.8* locus (*Nav1.8*^{*Cre*/Cre} mice) ¹⁸. Diphteria toxin (DT) treatment in *Nav1.8*^{*Cre*/+} GINIP^{*flx*/+} (hereafter called GINIP-DTR mice) at 4 weeks of age allowed the specific ablation of GINIP⁺ sensory neurons ¹⁶. *Nav1.8*^{*Crel*+} GINIP^{+/+} mice treated with DT were used as littermate controls throughout.

The ears of GINIP-DTR mice and littermate controls were exposed to UV irradiation 4 weeks after DT treatment. Tissue inflammation and damage was evaluated by measuring ear thickness every 2-3 days during 35 days (**data not shown**). We observed a strong inflammatory phase in both group between day 5 and day 10 post-UV exposition. Ear thickness was significantly increased in GINIP-DTR mice compared to control littermates starting at day 5 and until day 35 post-UV (**data not shown**). The peak of inflammation occurred at day 7 with a variation in ear thickness reaching 95µm (+/-11µm) in control mice versus 160µm (+/-35µm) in GINIP-DTR mice. The following recovery phase lasted 20 more days in the control group and more than 30 days in GINIP-DTR mice in which the ear thickness poorly reduced over time (**data not shown**). We then performed anatomo-pathology analysis at 14 and 35 days post-UV to better characterize the nature and extent of tissue damage in the two groups of mice (**data not shown**). Measuring the level of leukocyte infiltration (inflammation; **data not shown**), the thickening of the epidermis **(data not shown)** and collagen fiber expansion (fibrosis; **data not shown**), both groups reached a maximal score by day 14 suggesting massive tissue damages induced by UV-irradiation in both groups (**data not shown**). However, at day 35 leukocyte infiltration, epidermal thickness and fibrotic score were higher in GINIP-DTR mice compared to control littermates (**data not shown**). Accordingly, the cumulative score decreased significantly between day 14 and 35 in the control group but not in the GINIP-DTR mice group **(data not shown),** suggesting a defect in tissue remodeling in mice depleted for GINIP⁺ neurons. Further histological analysis of the ears at day 35 **(data not shown),** clearly showed a strong infiltration of collagen fibers throughout the whole dermis leading to the complete loss of the cartilage structure in ears of GINIP-DTR mice **(data not shown)** while the ears of control mice showed signs of fibrotic recovery (**data not shown**).

As tissue-resident macrophages and infiltrating monocytes are known to be central player in tissue remodeling and fibrosis, we analyzed the response of these cells in the skin of UV-exposed ears of GINIP-DTR and control littermates by flow cytometry. Excluding dendritic cells (CD11c⁺ MHC-II⁺), granulocytes (CD11b⁺ Ly6G⁺) and eosinophils (CD11b⁺ Siglec F⁺ CD24⁺) the monocyte/macrophage compartment, characterized by the double expression of CD11b and F480 **(data not shown),** can be further on separated on two subsets based on the expression of CCR2 and CD64 (**data not shown**). The CCR2⁺ population has been previously related to monocytes giving rise to monocytes-derived macrophages characterized by their high expression of MHC-II ^{19 20}. CD64 is commonly used to identify the tissue-resident macrophage populations²¹. Compared to control mice, GINIP-DTR mice exposed to UV irradiation presented a more persistent population of CCR2⁺ cells at day 14 **(data not shown)** as well as higher numbers of MHC-II⁺ macrophages between days 14 and 35 after UV irradiation (**data not shown**). Altogether, these data suggest that in the absence of GINIP⁺ neurons, the skin is more susceptible to UV-induced fibrosis and monocyte-derived cell infiltration.

GINIP is expressed in two distinct subsets of Ret⁺, non-peptidergic sensory neurons that can be distinguished through their differential staining with isolectin-B4 (IB4) and expression of TAFA4 ¹⁷. To delineate the underlying mechanisms leading to the strong skin damage observed in GINIP-DTR mice exposed to UV irradiation, we investigated by confocal microscopy on C3 DRGs which subsets of GINIP⁺ sensory neurons were reacting to UV exposure using a newly generated anti-TAFA4 monoclonal antibody, ATF3 and IB4 staining. The translocation of ATF3 was observed mostly in the nucleus of the IB4⁻ TAFA4⁺ subset (**data not shown**), which represents C-LTMR ^{17,22}. TAFA4 molecule, a marker of this subset, is a chemokine-like protein ²³ known to modulates injury-induced mechanical and chemical pain hypersensitivity in mice ²². Recombinant TAFA4 was shown to be involved in human macrophage chemotaxis *in vitro* ²⁴. However, its potential role on myeloid cell recruitment or activation *in vivo* is unknown. Therefore, we investigated if TAFA4 molecule could be involved in GINIP-DTR phenotype, by repeating UV-induced skin inflammation protocol on *Tafa4*-KO mice ²². The monitoring of ears thickness in *Tafa4*-KO and control littermates, showed an inflammatory phase culminating for both control and *Tafa4*-KO mice between day 7 and day 10 post-UV treatment (**data not shown**). During the recovery phase that followed, the ear thickness was progressively resolved by day 35 in control mice. However, the ear thickness was significantly higher in *Tafa-4KO* compared to control mice from day 18 to 35 suggesting a defect in resolving skin inflammation and/or tissue remodeling in the absence of TAFA4.

To further characterize the phenotype of *Tafa4*-KO mice before and after UV-induced skin damage, we performed an anatomo-pathology analysis on their ear skin (**data not shown**). At steady state, without exposure to UV **(data not shown),** the skin of *Tafa4*-KO and littermate control mice were similar without any sign of inflammation. After UV-irradiation, the nature and extent of tissue damage were similar in the two groups of mice between day 7 and 21 post-UV (**data not shown**). In contrast, at day 35, as observed in GINIP-DTR mice, the scores measuring leukocytes infiltration **(data not shown),** epidermal thickness **(data not shown)** and collagen deposition **(data not shown)** were higher in *Tafa4*-KO mice than in littermates WT controls, with a cumulative score of 5.7 +/- 2.2 compared to 8 +/- 1.5 for control and *Tafa4*-KO mice respectively (**data not shown**). At this time point *Tafa4*-KO mice thus presented a persistent fibrosis of the dermis compared to the controls group. Consistent with this observation, Picro Sirius Red staining and histological analysis revealed excessive type 1 collagen deposition and persistence of unresolved fibrotic scars within the dermis of *Tafa4*-KO mice compared to littermate controls at day 35 (**data not shown**). Finally, flow cytometry analysis of skin cells in the ears of *Tafa4*-KO mice after UV showed, as seen in GINIP-DTR mice, the persistence of CCR2⁺ cells at day 14 **(data not shown)** and the presence of more abundant MHC-II⁺ macrophages at day 35 (**data not shown**). Therefore, *Tafa4*-KO mice phenocopy, at least partially, the fibrotic pathology and the changes in myeloid cell response observed in GINIP-DTR mice. Altogether these data suggest that the neuropeptide TAFA4 produced by a subset of GINIP⁺ neurons regulates myeloid cell responses to skin injury after UV irradiation.

Our data suggest a correlation between unresolved dermal fibrosis and the persistence of monocyte-derived cells within the skin of *Tafa4*-KO mice upon UV exposure. We further characterized the phenotype of macrophage subsets of the dermis using flow cytometry analysis (**data not shown**). As tissue repair is associated with functions of alternatively activated macrophage, we used CD206 marker to identify them in the dermis. We observed that most CCR2⁻ CD64^{high} macrophages were positive for this marker **(data not shown)** suggesting that CD206 also highlights tissue-resident macrophages of the dermis (**data not shown**). Analyzing the skin throughout the lifespan of WT mice using this gating strategy, we observed that CD206 was already expressed before birth, at E17.5 of embryonic development (**data not shown**) suggesting that CD206 marks long-lived, embryonic-derived tissue-resident macrophages of the dermis ²⁵ The CCR2⁺ population mostly consists in infiltrating monocytes (R1; Ly6C⁺, MHC-II⁻), inflammatory monocytes (R2; Ly6C⁺ MHC-II⁺) and monocyte-derived macrophages (R3; Ly6C⁻MHC-II⁺) as observed here **(data not shown)** and in previous reports ²⁰. Indeed, this CCR2⁺ population is absent in embryonic dermis and only emerged within the first week after birth suggesting its relationship with bone marrow hematopoiesis (**data not shown**). Further analysis revealed that the CD206⁺ population contains two subsets of macrophages based on the expression or not of MHC-II (R4: MHC-II⁺ and R5: MHC-II⁻; **data not shown**). Using this gating strategy, we analyzed throughout the time course following UV exposure the dynamic of monocyte and macrophage subsets within the skin. UV treatment induced a massive recruitment of circulating monocytes, in both *Tafa4*-KO and littermate control mice, which culminated by day 5 and slowly decreased until day 23 (**data not shown**). No differences, in term of monocytes recruitment, were observed between the two groups. It is now well accepted that infiltrating monocytes progressively downregulate the expression of Ly6C and upregulate the expression of MHC-II to become inflammatory monocytes (**data not shown**). These inflammatory monocytes are known to produce II,-1-β and TNF-α as well as iNOS responsible for the production of reactive oxygen species (ROS) and can either give rise to Tip-dendritic cell or monocytes-derived macrophages depending on the tissue context ²⁶. Between day 14 to day 30 following UV irradiation, we observed an extended persistency of inflammatory monocytes within the dermis of *Tafa4*-KO mice compared to control littermates, suggesting that the absence of TAFA4 favors infiltrating monocyte to differentiate towards an inflammatory phenotype (**data not shown**). The monocyte-derived macrophage population (Ly6C⁻ MHC-II⁺) also expanded significantly faster in *Tafa4*-KO than in control mice, at least until day 25 (**data not shown**). The resident dermal macrophages (R5), known to emerge before birth from embryonic precursors seeding the skin during organogenesis ²⁷, are long-lived cells maintained continually through local self-renewal without input from bone marrow circulating precursors ²⁸. However, several tissue-resident macrophage populations such as those of the gut, the heart as well as the dermis can still receive, although at a slow rate, some contribution from circulating monocytes to sustain their macrophage pool ^{29 30 20}. After UV treatment, the two subsets (MHC-II⁻ and MHC-II⁺) of resident CD206+ dermal macrophages rapidly expanded until at least day 25 in both groups of mice (**data not shown**). Importantly, the number of dermal macrophages was higher in the skin of *Tafa4*-KO mice from day 7 to day 25. Combining the different macrophage populations identified in the dermis, the overall macrophage pool strongly expanded until day 35 and remarkably more rapidly in *Tafa4*-KO mice compared to control littermates (**data not shown**). This set of data first shows that UV irradiation triggers the recruitment of a massive number of monocytes from the circulation to provide additional macrophages locally probably supporting the dermal resident pool functions. These data show that TAFA4 is required for the control of dermal macrophage response triggered by UV exposure. Moreover, they highly suggest that the absence of TAFA4 leads to an excessive local expansion of dermal macrophage potentially responsible for the unresolved skin fibrosis phenotype.

As resident macrophages and monocyte-derived macrophages could have distinct function and contribution to the observed phenotype, we generated a series of bone marrow (BM) chimera in which the ears skin and their resident cells are protected from radiation by a lead shield (Ajami et al., 2007; **data not shown**). Monocytes circulation from the bone marrow to inflammed tissues highly relies on the CCR2/CCL2 axis ³¹. To get insight on the monocyte contribution to the different macrophage subsets, we followed the expansion, in absolute number, of each population described earlier, 7 days post-UV, either in WT BM chimera mice **(data not shown)** or in WT chimera reconstituted with BM from CCR2-KO mice (**data not shown**). In CCR2-KO BM chimera mice, monocytes, inflammatory monocytes and monocytes-derived macrophages could not reach the inflammed dermis after UV treatment and could not contribute to the necessary expansion of these populations in the inflamed dermis (**data not shown**). As expected, the resident dermal macrophage population **(data not shown)** however could still expand independently from circulating precursors although with a noticed reduction compared to WT BM chimera mice. Interestingly, the MHCII+ CD206+ macrophage subset was rather highly dependent from circulating precursors. This suggests that this population could represent an intermediate subset bridging circulating monocytes and the long-lived *bona fide* resident dermal macrophage population (**data not shown**).

To further investigate how the microenvironment impacts monocytes fate in the absence of TAFA4, we reconstituted CD45.2 WT or *Tafa4*-KO irradiated mice with CD45.1 BM. In these conditions infiltrating monocyte fate can be traced based on CD45.1 expression while resident dermal macrophages (R5) could be identified through CD45.2 expression. Resident macrophages expanded drastically after UV irradiation and even faster in *Tafa4*-KO mice compared to controls littermates **(data not shown)** in both WT and Tafa4-KO chimera mice unexposed to UV, five weeks after reconstitution, the contribution of WT CD45.1 BM to the resident dermal macrophage pool (R5) remained below 25% (**data not shown**). As expected, the frequency of CD45.1⁺ brain microglia, which remains independent from circulating monocytes reached less than 3%, while circulating monocytes and granulocytes reached 95% (**data not shown**). These data suggest a slow but consistent turnover of dermal-resident macrophages. In contrast, upon exposure to UV, the contribution of CD45.1+ BM circulating precursors to dermal macrophages reached 55% (+/- 8%) in WT versus 74% (+/- 9%) *Tafa4-*KO recipient mice, 14 days after UV treatment. Two alternative explanations could underlie these data. Resident dermal macrophages could be UV sensitive, leading, after their elimination for example through necroptosis mechanisms ³² to their replacement by circulating monocytes. Alternatively, the resident dermal macrophage pool could remain stable but their relative frequency would be drowned following a massive expansion of monocyte-derived cells which can differentiate into CD206⁺ MHCII+ intermediate subset as observed earlier (**data not shown,** UV groups). The analysis of the same cell subsets at day 35 post-UV treatment supports the second hypothesis as the chimerism of dermal macrophages returned to 41% +/-12% in WT recipient and 55% +/-13% in *Tafa4*-KO recipient (**data not shown**). Three months after UV exposure, the chimerism of dermal resident macrophages decreased to 34% +/- 7% in WT recipient while it increased to 63% +/- 12% in *Tafa4*-KO recipient mice, respectively (**data not shown**). These data suggest that monocytes differentiation towards resident dermal macrophages, observed in WT recipient mice is transient and that *bona fide* dermal macrophage expand during the recovery phase to take over their initial niche. In addition, our results suggest that in the absence of TAFA4, monocyte-derived dermal macrophages remain longer into the dermis and potentially become long-lived resident cells. The origin of dermal macrophages in the *Tafa4*-KO versus WT mice are thus different suggesting potential different functionality.

To further understand the mechanism by which TAFA4 could regulate myeloid cell response, we tested if recombinant TAFA4 was able to directly modulate their function *in vitro.* In contrast to a previous report ²⁴, we were not able to detect any chemotactic activity of TAFA4 on mouse macrophages (**data not shown**). However, we found that the transcriptomic profile of IL-4 primed BM-derived macrophages was modified in the presence of TAFA4. The expression of transcription factors encoding genes, such as IRF5 and IRF8, involved in developmental maturation of macrophages and polarisation ^{33,34} were upregulated by increasing concentration of TAFA4 (**data not shown**). Moreover, the gene *Nr4a1,* required for the regulation of macrophage activation and apoptosis as well as for monocytes differentiation and Ly6C^{lo} monocyte survival ³⁵⁻³⁷ was also upregulated by TAFA4 (**data not shown**). The mRNA encoding TGF-β was also upregulated in response to TAFA4 (**data not shown**). TGF-β, besides being a central factor for the resolution of inflammation, has been recently implicated in the maintenance of several resident macrophage as well as in monocyte recruitment and differentiation^{38,39}. These data suggest that TAFA4 can directly act on macrophages and activate regulatory pathways involved in key functions of macrophages and monocytes. Finally, thioglycolate-induced inflammatory macrophages, treated in vitro with lipopolysaccharide ⁴⁰ downregulated, in response to additional TAFA4, the Receptor-interacting protein kinase 3 (RIPK3), a gene involved in necroptosis suggesting that TAFA4 could protect macrophages from death induced by excessive inflammation ⁴¹ (**data not shown**).

These *in vitro* data suggest that TAFA4 could directly modulate myeloid cell homeostasis. Its overall absence in *Tafa4*-KO mice could favor tissue-resident macrophage replacement under inflammatory condition. Consistent with a potential role of TAFA4 in inducing an anti-inflammatory transcriptomic profile in macrophages, we found more Ly6C⁺ MHCII⁺ inflammatory monocytes producing II,-1-β and TNF-α in *Tafa4*-KO mice compared to littermate controls *in vivo* 7 days after UV exposure (**data not shown**). We showed that inflammatory cytokines and chemokines production, such as TFN-α, IL-1b, IL-6, CXCL1, CCL4 or CCL2 production, are extended in TAFA4-KO mice in total skin after 7 or 10 days of UV irradiation (**Data not shown**). The expression of IL-10 is upregulated in purified macrophage in WT mice after UV irradiation, but not in TAFA4 KO-mice. An ex vivo analysis of IL-10 production in macrophage subsets (Tim4+ Mac, DN Mac and MCHII+ Mac) was proceed. We found that two subsets of dermal macrophages, Tim4+ Mac and DN mac, upregulate IL-10 production in vivo following UV irradiation of the skin in WT mice but not in TAFA4 KO mice (**data not shown**). This suggests that TAFA4 could not only regulate resident cell biology but could also inhibit inflammatory functions of newly recruited monocytes into tissues.

To confirm the anti-inflammatory potential of TAFA4, we performed intradermal injections of TAFA4 (100nM, 50ul) or saline in the ears of *Tafa4*-KO mice every 2 days starting the day of UV treatment during 8 days. The follow up of ear thickness over time revealed a reduction of the inflammatory phase in the Tafa4-KO mice treated with TAFA4 (110µm +/-11µm) compared to *Tafa4*-KO or control mice treated with saline (145uM+/- 23µm and 135+/- 27µm respectively) at day 10 (**Fig. 1A**). The reduction of ear thickness in TAFA4-treated versus saline-injected *Tafa4*-KO mice was also significant between day 14 and 19 post-UV exposure (**Fig. 1A**). However, at later time points, the ear thickness returned to the level observed in the *Tafa4*-KO control group treated with saline suggesting that the presence of TAFA4 is required during the whole process of tissue repair. We then analyzed if the reduction in ear thickness of *Tafa4*-KO mice treated with TAFA4 protein was associated with modifications in myeloid cell responses. Consistent with the phenotype described earlier, in mice injected with saline, a clear increase in the number of CCR2⁺ macrophages was observed in the ears of *Tafa4*-KO compared to control littermates, 7 days after UV irradiation. In contrast, *Tafa4*-KO mice treated with TAFA4 showed a rescue of this phenotype with CCR2+ macrophages in the ear skin (**Figure 1B**). In addition, the local injection of TAFA4 also reduced the number of MHC-II⁺ macrophages in the ears of *Tafa4*-KO mice 7 days after UV irradiation (**Fig. 1C**)**.** Therefore, injection of TAFA4 in injured skin can restore the impaired response observed in *Tafa4*-KO mice *in vivo* suggesting that such treatment may have a potential interest in some pathological conditions associated with skin inflammatory diseases.

Altogether, these data show that TAFA4, a molecule expressed by a subset of GINIP⁺ sensory neurons innervating the skin is required for the resolution of skin inflammation and fibrosis upon exposure to UV irradiation. Our results also suggest that treatment with TAFA4 *in vitro* and *in vivo* can promote an anti-inflammatory and pro-repair myeloid cell responses.

Primary sensory neurons are heterogeneous by numerous molecular criteria⁷. The variety of functional outcomes observed upon sensory neuron deficiencies could be explained by the complexity and diversity of sensory neurons subtypes. However the functional significance of this remarkable heterogeneity is just emerging. Previous studies have shown that sensory neurons involved in pain sensitivity can modulate some aspects of skin inflammatory immune responses². However, most of them focused on the role of peptidergic subsets and in particular on the role of the neuropeptide CGRP that has been described to have mainly pro-inflammatory effects^{3,4}. Here we describe that a group of non peptidergic neurons expressing the marker GINIP are required to down-regulate inflammatory processes and to modulate the myeloid cell response. This control is required to promote tissue repair in the context of skin exposure to UV irradiation. We identified TAFA4 a factor produced by GINIP⁺ C-LTMR has a major factor involved in this regulation. However, the skin inflammatory phenotype in *Tafa4*-KO mice is less pronounced than in GINIP-DTR mice (**data not shown**) suggesting that other mediators expressed by GINIP⁺ neurons may have additional regulatory functions. This study reveals new mechanisms of neuro-immune regulations and paves the way for future investigations analyzing the role of this novel regulatory pathway in other skin inflammatory diseases.

### REFERENCES:

Throughout this application, various references describe the state of the art to which this disclosure pertains.
1 Guilliams, M. & Scott, C. L. Does niche competition determine the origin of tissue-resident macrophages? Nat Rev Immunol 17, 451-460, doi: 10.1038/nri.2017.42 (2017).
2 Pinho-Ribeiro, F. A., Verri, W. A., Jr. & Chiu, I. M. Nociceptor Sensory Neuron-Immune Interactions in Pain and Inflammation. Trends Immunol 38, 5-19, doi:10.1016/j.it.2016.10.001 (2017).
3 Kashem, S. W. et al. Nociceptive Sensory Fibers Drive Interleukin-23 Production from CD301b+ Dermal Dendritic Cells and Drive Protective Cutaneous Immunity. Immunity 43, 515-526, doi:10.1016/j.immuni.2015.08.016 (2015).
4 Riol-Blanco, L. et al. Nociceptive sensory neurons drive interleukin-23-mediated psoriasiform skin inflammation. Nature 510, 157-161, doi:10.1038/nature13199 (2014).
5 Chiu, I. M. et al. Bacteria activate sensory neurons that modulate pain and inflammation. Nature 501, 52-57, doi: 10. 10 38/nature12479 (2013).
6 Pinho-Ribeiro, F. A. et al. Blocking Neuronal Signaling to Immune Cells Treats Streptococcal Invasive Infection. Cell 173, 1083-1097 e1022, doi:10.1016/j.cell.2018.04.006 (2018).
7 Usoskin, D. et al. Unbiased classification of sensory neuron types by large-scale single-cell RNA sequencing. Nat Neurosci 18, 145-153, doi:10.1038/nn.3881 (2015).
8 Merad, M. et al. Langerhans cells renew in the skin throughout life under steady-state conditions. Nat Immunol 3, 1135-1141. (2002).
9 Ajami, B., Bennett, J. L., Krieger, C., Tetzlaff, W. & Rossi, F. M. Local self-renewal can sustain CNS microglia maintenance and function throughout adult life. Nat Neurosci 10, 1538-1543, doi:nn2014 [pii] 10.1038/nn2014 (2007).
10 Wynn, T. A. & Vannella, K. M. Macrophages in Tissue Repair, Regeneration, and Fibrosis. Immunity 44, 450-462, doi:10.1016/j.immuni.2016.02.015 (2016).
11 Lavin, Y. et al. Tissue-resident macrophage enhancer landscapes are shaped by the local microenvironment. Cell 159, 1312-1326, doi:10.1016/j.cell.2014.11.018 (2014).
12 Gosselin, D. et al. Environment drives selection and function of enhancers controlling tissue-specific macrophage identities. Cell 159, 1327-1340, doi:10.1016/j.cell.2014.11.023 (2014).
13 Okabe, Y. & Medzhitov, R. How the immune system spots tumors. Elife 3, e04476, doi:10.7554/eLife.04476 (2014).
14 Lopes, D. M. & McMahon, S. B. Ultraviolet Radiation on the Skin: A Painful Experience? CNS Neurosci Ther 22, 118-126, doi:10.1111/cns.12444 (2016).
15 Braz, J. M. & Basbaum, A. I. Differential ATF3 expression in dorsal root ganglion neurons reveals the profile of primary afferents engaged by diverse noxious chemical stimuli. Pain 150, 290-301, doi:10.1016/j.pain.2010.05.005 (2010).
16 Urien, L. et al. Genetic ablation of GINIP-expressing primary sensory neurons strongly impairs Formalin-evoked pain. Sci Rep 7, 43493, doi:10.1038/srep43493 (2017).
17 Gaillard, S. et al. GINIP, a Galphai-interacting protein, functions as a key modulator of peripheral GABAB receptor-mediated analgesia. Neuron 84, 123-136, doi:10.1016/j.neuron.2014.08.056 (2014).
18 Abrahamsen, B. et al. The cell and molecular basis of mechanical, cold, and inflammatory pain. Science 321, 702-705, doi: 10. 1 126/science. 1156916 (2008).
19 Jakubzick, C. et al. Minimal differentiation of classical monocytes as they survey steady-state tissues and transport antigen to lymph nodes. Immunity 39, 599-610, doi:10.1016/j.immuni.2013.08.007 (2013).
20 Tamoutounour, S. et al. Origins and functional specialization of macrophages and of conventional and monocyte-derived dendritic cells in mouse skin. Immunity 39, 925-938, doi:10.1016/j.immuni.2013.10.004 (2013).
21 Gautier, E. L. et al. Gene-expression profiles and transcriptional regulatory pathways that underlie the identity and diversity of mouse tissue macrophages. Nat Immunol 13, 1118-1128, doi:10.1038/ni.2419 (2012).
22 Delfini, M. C. et al. TAFA4, a chemokine-like protein, modulates injury-induced mechanical and chemical pain hypersensitivity in mice. Cell Rep 5, 378-388, doi:10.1016/j.celrep.2013.09.013 (2013).
23 Tom Tang, Y. et al. TAFA: a novel secreted family with conserved cysteine residues and restricted expression in the brain. Genomics 83, 727-734, doi:10.1016/j.ygeno.2003.10.006 (2004).
24 Wang, W. et al. FAM19A4 is a novel cytokine ligand of formyl peptide receptor 1 (FPR1) and is able to promote the migration and phagocytosis of macrophages. Cellular & molecular immunology 12, 615-624, doi:10.1038/cmi.2014.61 (2015).
25 Hoeffel, G. et al. C-Myb(+) erythro-myeloid progenitor-derived fetal monocytes give rise to adult tissue-resident macrophages. Immunity 42, 665-678, doi:10.1016/j.immuni.2015.03.011 (2015).
26 Serbina, N. V., Salazar-Mather, T. P., Biron, C. A., Kuziel, W. A. & Pamer, E. G. TNF/iNOS-producing dendritic cells mediate innate immune defense against bacterial infection. Immunity 19, 59-70 (2003).
27 Hoeffel, G. & Ginhoux, F. Fetal monocytes and the origins of tissue-resident macrophages. Cell Immunol, doi:10.1016/j.cellimm.2018.01.001 (2018).
28 Ginhoux, F. & Jung, S. Monocytes and macrophages: developmental pathways and tissue homeostasis. Nat Rev Immunol 14, 392-404, doi:10.1038/nri3671 (2014).
29 Bain, C. C. et al. Constant replenishment from circulating monocytes maintains the macrophage pool in the intestine of adult mice. Nat Immunol 15, 929-937, doi:10.1038/ni.2967 (2014).
30 Molawi, K. et al. Progressive replacement of embryo-derived cardiac macrophages with age. J Exp Med 211, 2151-2158, doi:10.1084/jem.20140639 (2014).
31 Serbina, N. V. & Pamer, E. G. Monocyte emigration from bone marrow during bacterial infection requires signals mediated by chemokine receptor CCR2. Nat Immunol 7, 311 -317, doi: 10.1038/ni 1309 (2006).
32 Bleriot, C. et al. Liver-resident macrophage necroptosis orchestrates type 1 microbicidal inflammation and type-2-mediated tissue repair during bacterial infection. Immunity 42, 145-158, doi:10.1016/j.immuni.2014.12.020 (2015).
33 Lawrence, T. & Natoli, G. Transcriptional regulation of macrophage polarization: enabling diversity with identity. Nat Rev Immunol 11, 750-761, doi:10.1038/nri3088 (2011).
34 Hagemeyer, N. et al. Transcriptome-based profiling of yolk sac-derived macrophages reveals a role for Irf8 in macrophage maturation. EMBO J 35, 1730-1744, doi:10.15252/embj.201693801 (2016).
35 Palumbo-Zerr, K. et al. Orphan nuclear receptor NR4A1 regulates transforming growth factor-beta signaling and fibrosis. Nat Med 21, 150-158, doi:10.1038/nm.3777 (2015).
36 Hanna, R. N. et al. The transcription factor NR4A1 (Nur77) controls bone marrow differentiation and the survival of Ly6C- monocytes. Nat Immunol 12, 778-785, doi:10.1038/ni.2063 (2011).
37 Thomas, G. D. et al. Deleting an Nr4a1 Super-Enhancer Subdomain Ablates Ly6Clow Monocytes while Preserving Macrophage Gene Function. Immunity 45, 975-987, doi:10.1016/j.immuni.2016.10.011 (2016).
38 Yu, X. et al. The Cytokine TGF-beta Promotes the Development and Homeostasis of Alveolar Macrophages. Immunity 47, 903-912 e904, doi:10.1016/j.immuni.2017.10.007 (2017).
39 Lund, H. et al. Fatal demyelinating disease is induced by monocyte-derived macrophages in the absence of TGF-beta signaling. Nat Immunol 19, 1-7, doi:10.1038/s41590-018-0091-5 (2018).
40 Bogunovic, M. et al. Identification of a radio-resistant and cycling dermal dendritic cell population in mice and men. J Exp Med 203, 2627-2638, doi:10.1084/jem.20060667 (2006).
41 Vanden Berghe, T., Linkermann, A., Jouan-Lanhouet, S., Walczak, H. & Vandenabeele, P. Regulated necrosis: the expanding network of non-apoptotic cell death pathways. Nat Rev Mol Cell Biol 15, 135-147, doi:10.1038/nrm3737 (2014).
42 Greter, M. et al. Stroma-derived interleukin-34 controls the development and maintenance of langerhans cells and the maintenance of microglia. Immunity 37, 1050-1060, doi:10.1016/j.immuni.2012.11.001 (2012).

## Claims

1. A TAFA4 polypeptide or a nucleic acid molecule encoding thereof for use for reducing skin inflammation in a subject in need thereof.

2. The TAFA4 polypeptide or a nucleic acid molecule for use according to claim 1 wherein the subject suffers from an inflammatory skin disease.

3. The TAFA4 polypeptide or a nucleic acid molecule for use according to claim 1 wherein the subject suffers from an inflammatory skin disease selected from the group consisting of acne, rosacea, folliculitis, perioral dermatitis, photodamage, skin aging, psoriasis, ichtiosis, chronic wounds, bed sores, keratosis piralis, scars, including surgical and acne scars, sebaceous cysts, inflammatory dermatoses, post inflammatory hyperpigmentation, xerosis, pruritis, lichen planus, nodular prurigo, eczema, and miliaria.

4. The TAFA4 polypeptide or a nucleic acid molecule for use according to claim 1 wherein the subject suffers from an inflammatory skin disease selected from the group consisting of scleroderma, atopic dermatitis, nephrogenic fibrosing dermopathy, mixed connective tissue disease, scleromyxedema, scleredema, keloid, sclerodactyly, or eosinophilic fasciitis.

5. The TAFA4 polypeptide or a nucleic acid molecule for use according to claim 1 wherein the subject suffers from photodermatitis.

6. The TAFA4 polypeptide or a nucleic acid molecule for use according to claim 1 wherein the subject suffers from chronic wound.

7. The TAFA4 polypeptide or a nucleic acid molecule for use according to claim 1 wherein the subject suffers from venous stasis ulcers or diabetic foot ulcers.

8. The TAFA4 polypeptide or a nucleic acid molecule for use according to claim 1 for use for treating chronic wound in subject suffering from sickle-cell disease or chronic wound in elderly.

9. The TAFA4 polypeptide or a nucleic acid molecule for use according to claim 1 for use for preventing skin fibrosis.

10. The TAFA4 polypeptide or a nucleic acid molecule for use according to claim 1 wherein the TAFA4 polypeptide comprises a sequence having at least 90% sequence identity to the sequence as set forth in SEQ ID NO: 1.

11. The TAFA4 polypeptide or a nucleic acid molecule for use according to claim 1 wherein the TAFA4 polypeptide is fused to an immunoglobulin constant domain to constitute an immunoadhesin.

12. The TAFA4 polypeptide or a nucleic acid molecule for use according to claim 1 wherein the nucleic acid molecule is included in a suitable vector, such as viral vector (e.g. AAV).

13. The TAFA4 polypeptide or a nucleic acid molecule for use according to claim 1 wherein the TAFA4 polypeptide or the nucleic acid molecule encoding thereof are formulated for topical administration.

14. The TAFA4 polypeptide or a nucleic acid molecule for use according to claim 13 wherein the topical administration for cutaneous treatment is accomplished via a transdermal device or patch device.

15. The TAFA4 polypeptide or a nucleic acid molecule for use according to claim 1 wherein he TAFA4 polypeptide or the nucleic acid molecule encoding thereof the TAFA4 polypeptide is administered with another active agent such as a glucocorticoid.

## Patentansprüche

1. TAFA4-Polypeptid oder ein Nukleinsäuremolekül, das dafür codiert, zur Verwendung zur Verringerung von Hautentzündung bei einem Subjekt, das dies benötigt.

2. TAFA4-Polypeptid oder ein Nukleinsäuremolekül zur Verwendung nach Anspruch 1, wobei das Subjekt an einer entzündlichen Hauterkrankung leidet.

3. TAFA4-Polypeptid oder ein Nukleinsäuremolekül zur Verwendung nach Anspruch 1, wobei das Subjekt an einer entzündlichen Hauterkrankung leidet, die ausgewählt ist aus der Gruppe bestehend aus Akne, Rosacea, Follikulitis, perioraler Dermatitis, Lichtschaden, Hautalterung, Psoriasis, Ichtyose, chronischen Wunden, Druckgeschwüren, Keratosis piralis, Narben, beinhaltend chirurgische und Aknenarben, Talgzysten, entzündlichen Dermatosen, postentzündlicher Hyperpigmentierung, Xerose, Pruritis, Lichen planus, nodulärer Prurigo, Ekzem und Miliaria.

4. TAFA4-Polypeptid oder ein Nukleinsäuremolekül zur Verwendung nach Anspruch 1, wobei das Subjekt an einer entzündlichen Hauterkrankung leidet, die ausgewählt ist aus der Gruppe bestehend aus Skleroderm, atopischer Dermatitis, nephrogener fibrosierender Dermopathie, gemischter Bindegewebeerkrankung, Skleromyxödem, Sklerodermie, Keloid, Sklerodaktylie oder eosinophiler Fasciitis.

5. TAFA4-Polypeptid oder ein Nukleinsäuremolekül zur Verwendung nach Anspruch 1, wobei das Subjekt an Photodermatitis leidet.

6. TAFA4-Polypeptid oder ein Nukleinsäuremolekül zur Verwendung nach Anspruch 1, wobei das Subjekt an einer chronischen Wunde leidet.

7. TAFA4-Polypeptid oder ein Nukleinsäuremolekül zur Verwendung nach Anspruch 1, wobei das Subjekt an Venostasegeschwüren oder diabetischen Fußgeschwüren leidet.

8. TAFA4-Polypeptid oder ein Nukleinsäuremolekül zur Verwendung nach Anspruch 1 zur Verwendung zur Behandlung einer chronischen Wunde bei einem Subjekt, das an Sichelzellenkrankheit leidet, oder von chronischen Wunden bei älteren Patienten.

9. TAFA4-Polypeptid oder ein Nukleinsäuremolekül zur Verwendung nach Anspruch 1 zur Verwendung zur Verhinderung von Hautfibrose.

10. TAFA4-Polypeptid oder ein Nukleinsäuremolekül zur Verwendung nach Anspruch 1, wobei das TAFA4-Polypeptid eine Sequenz mit mindestens 90% Sequenzidentität mit der in SEQ ID NR: 1 angegebenen Sequenz umfasst.

11. TAFA4-Polypeptid oder ein Nukleinsäuremolekül zur Verwendung nach Anspruch 1, wobei das TAFA4-Polypeptid mit einer konstanten Domäne eines Immunglobulins fusioniert ist, um ein Immunoadhäsin zu bilden.

12. TAFA4-Polypeptid oder ein Nukleinsäuremolekül zur Verwendung nach Anspruch 1, wobei das Nukleinsäuremolekül in einem geeigneten Vektor wie einem viralen Vektor (z.B. AAV) beinhaltet ist.

13. TAFA4-Polypeptid oder ein Nukleinsäuremolekül zur Verwendung nach Anspruch 1, wobei das TAFA4-Polypeptid oder das Nukleinsäuremolekül, das dafür codiert, zur topischen Verabreichung formuliert sind.

14. TAFA4-Polypeptid oder ein Nukleinsäuremolekül zur Verwendung nach Anspruch 13, wobei die topische Verabreichung zur kutanen Behandlung über eine transdermale Vorrichtung oder eine Patch-Vorrichtung erfolgt.

15. TAFA4-Polypeptid oder ein Nukleinsäuremolekül zur Verwendung nach Anspruch 1, wobei das TAFA4-Polypeptid oder das Nukleinsäuremolekül, das dafür codiert, das TAFA4-Polypeptid mit einem anderen Wirkstoff wie einem Glucokortikoid verabreicht wird.

## Revendications

1. Polypeptide TAFA4 ou molécule d'acide nucléique codant pour celui-ci pour utilisation dans la réduction de l'inflammation cutanée chez un sujet en ayant besoin.

2. Polypeptide TAFA4 ou molécule d'acide nucléique pour utilisation selon la revendication 1, dans lequel le sujet souffre d'une maladie inflammatoire de la peau.

3. Polypeptide TAFA4 ou molécule d'acide nucléique pour utilisation selon la revendication 1, dans lequel le sujet souffre d'une maladie inflammatoire de la peau sélectionnée parmi le groupe consistant en l'acné, la rosacée, la folliculite, la dermite périorale, le photovieillissement, le vieillissement cutané, le psoriasis, l'ichtyose, les plaies chroniques, les escarres, la kératose pilaire, les cicatrices, comprenant les cicatrices chirurgicales et liées à l'acné, les kystes sébacés, les dermatoses inflammatoires, l'hyperpigmentation post-inflammatoire, la xérose, le prurit, le lichen plan, le prurigo nodulaire, l'eczéma, et la fièvre miliaire.

4. Polypeptide TAFA4 ou molécule d'acide nucléique pour utilisation selon la revendication 1, dans lequel le sujet souffre d'une maladie inflammatoire de la peau sélectionnée parmi le groupe consistant en la sclérodermie, la dermatite atopique, la dermopathie fibrosante néphrogénique, la connectivité mixte, le scléromyxoedème, le scléroedème, la chéloïde, la sclérodactylie, ou la fasciite à éosinophiles.

5. Polypeptide TAFA4 ou molécule d'acide nucléique pour utilisation selon la revendication 1, dans lequel le sujet souffre de photodermatose.

6. Polypeptide TAFA4 ou molécule d'acide nucléique pour utilisation selon la revendication 1, dans lequel le sujet souffre d'une plaie chronique.

7. Polypeptide TAFA4 ou molécule d'acide nucléique pour utilisation selon la revendication 1, dans lequel le sujet souffre d'ulcères de stase veineuse ou d'ulcères du pied diabétique.

8. Polypeptide TAFA4 ou molécule d'acide nucléique pour utilisation selon la revendication 1, pour utilisation dans le traitement d'une plaie chronique chez un sujet souffrant de drépanocytose ou d'une plaie chronique chez les personnes âgées.

9. Polypeptide TAFA4 ou molécule d'acide nucléique pour utilisation selon la revendication 1, pour utilisation dans la prévention de la fibrose cutanée.

10. Polypeptide TAFA4 ou molécule d'acide nucléique pour utilisation selon la revendication 1, dans lequel le polypeptide TAFA4 comprend une séquence présentant au moins 90 % d'identité de séquence par rapport à la séquence telle que définie dans SEQ ID NO: 1.

11. Polypeptide TAFA4 ou molécule d'acide nucléique pour utilisation selon la revendication 1, dans lequel le polypeptide TAFA4 est fusionné à un domaine constant d'immunoglobuline pour constituer une immunoadhésine.

12. Polypeptide TAFA4 ou molécule d'acide nucléique pour utilisation selon la revendication 1, dans lequel la molécule d'acide nucléique est incluse dans un vecteur approprié, tel qu'un vecteur viral (par ex. un AAV).

13. Polypeptide TAFA4 ou molécule d'acide nucléique pour utilisation selon la revendication 1, dans lequel le polypeptide TAFA4 ou la molécule d'acide nucléique codant pour celui-ci sont formulé pour une administration topique.

14. Polypeptide TAFA4 ou molécule d'acide nucléique pour utilisation selon la revendication 13, dans lequel l'administration topique pour un traitement cutané s'effectue par l'intermédiaire d'un dispositif transdermique ou d'un dispositif de type patch.

15. Polypeptide TAFA4 ou molécule d'acide nucléique pour utilisation selon la revendication 1, dans lequel le polypeptide TAFA4 ou la molécule d'acide nucléique codant pour celui-ci, le polypeptide TAFA4 est administré avec un autre agent actif tel qu'un glucocorticoïde.
